# EUROPEAN PATENT SPECIFICATION

(11) **EP 2 197 783 B1**
(45) Date of publication and mention of the grant of the patent: **11.07.2018**
(21) Application number: 08835082.2
(22) Date of filing: 02.10.2008
(51) Int. Cl.: A61K 49/00, B82Y 5/00, A61K 49/08, A61K 49/14, A61K 49/18

(54) **COMPLEX OF CELL TRANSLOCATIONAL PEPTIDE AND MAGNETIC NANOPARTICLES AND USE THEREOF**
KOMPLEX AUS TRANSLOKATIONALEM ZELLPEPTID UND MAGNETNANOPARTIKELN UND SEINE VERWENDUNG
COMPLEXE CONSTITUÉ D'UN PEPTIDE DE TRANSLOCATION ET DE NANOPARTICULES MAGNÉTIQUES, ET UTILISATION D'UN TEL COMPLEXE

(30) Priority: 02.10.2007 KR 20070099511
(43) Date of publication of application: 23.06.2010
(73) Proprietor: Seoul National University Industry Foundation, Gwanak-gu Seoul 151-050 (KR)
(72) Inventor: PARK, Yoon-Jeong, Seoul 152-770 (KR); CHUNG, Chong-Pyoung, Seoul 138-170 (KR); YANG, Victor, C., Michigan 48109 (US); SUH, Jin Sook, Seoul 157-922 (KR)
(74) Representative: Hiebl, Inge Elisabeth
(86) International application number: PCT/KR2008/005815
(87) International publication number: WO 2009/045063

(56) References cited:
- WO-A2-2004/081188
- WO-A2-2005/107818
- WO-A2-2007/149062
- US-A1- 2006 251 726
- US-B2- 6 867 186
- US-B2- 7 019 113
- US-B2- 7 037 652
- FUNOVICS MARTIN ET AL: "Nanoparticles for the optical imaging of tumor E-selectin", NEOPLASIA, NEOPLASIA PRESS, ANN ARBOR, MI, US, vol. 7, no. 10, 1 October 2005 (2005-10-01), pages 904-911, XP009105232, ISSN: 1522-8002, DOI: 10.1593/NEO.05352
- MARCELO J KOGAN ET AL: "Peptides and metallic nanoparticles for biomedical applications", NANOMEDICINE, vol. 2, no. 3, 1 June 2007 (2007-06-01), pages 287-306, XP55104016, ISSN: 1743-5889, DOI: 10.2217/17435889.2.3.287
- JOSEPHSON L ET AL: "High-efficiency intracellular magnetic labeling with novel superparamagnetic-Tat peptide conjugates", BIOCONJUGATE CHEMISTRY, ACS, WASHINGTON, DC, US, vol. 10, no. 2, 1 February 1999 (1999-02-01), pages 186-191, XP002172120, ISSN: 1043-1802, DOI: 10.1021/BC980125H
- PARK YOON JEONG ET AL: "Nontoxic membrane translocation peptide from protamine, low molecular weight protamine (LMWP), for enhanced intracellular protein delivery: in vitro and in vivo study", THE FASEB JOURNAL, FEDERATION OF AMERICAN SOCIETIES FOR EXPERIMENTAL BIOLOGY, BETHESDA; US, vol. 19, no. 9, 1 July 2005 (2005-07-01), pages 1-20, XP002587876, ISSN: 0892-6638, DOI: 10.1096/FJ.04-2322FJE [retrieved on 2005-07-20]
- APOSTOLOPOULOS V ET AL: "Delivery of tumor associated antigens to antigen presenting cells using penetratin induces potent immune responses", VACCINE, ELSEVIER LTD, GB, vol. 24, no. 16, 12 April 2006 (2006-04-12), pages 3191-3202, XP028010607, ISSN: 0264-410X, DOI: 10.1016/J.VACCINE.2006.01.032 [retrieved on 2006-04-12]
- CHRISTIAENS B ET AL: "Enhancement of polymethacrylate-mediated gene delivery by Penetratin", EUROPEAN JOURNAL OF PHARMACEUTICAL SCIENCES, ELSEVIER, AMSTERDAM, NL, vol. 24, no. 5, 1 April 2005 (2005-04-01), pages 525-537, XP027803775, ISSN: 0928-0987 [retrieved on 2005-04-01]

## Description

### TECHNICAL FIELD

The present invention relates to a cell-penetrating peptide/fluorescence-labeled magnetic nanoparticle complex and the use thereof, and more particularly to cell-penetrating peptide/fluorescence-labeled magnetic nanoparticle complex in which the cell-penetrating peptide is chemically linked to the fluorescence-labeled magnetic nanoparticle, such that fluorescence-labeled magnetic nanoparticle can be stably introduced directly into cells without endocytosis, and a composition for cell imaging containing the complex.

### BACKGROUND ART

Molecular imaging is a rapidly developing discipline that visualizes various changes occurring at the molecular level in cells, that is, genetic expressions, biochemical events and biological changes. An existing medical imaging technique comprises producing image signals using the difference in nonspecific physical and chemical properties and clinically analyzing the image signals, whereas the molecular imaging technique is characterized by more specific images, because it uses image signals produced by action at the molecular and genetic levels. The use of this molecular imaging technique enables genetic changes, protein changes, metabolic changes, intracellular biological changes and the like to be imaged.

In existing molecular biological research methods for analyzing the position, level and period of expression of various genes in cells or tissues, the sacrifice of test animals or damage to the tissue of patients are necessary, because target tissues or cells must be collected through tissue biopsy. For this reason, when the existing research methods are used, it is impossible to carry out repeated experiments in one individual, because the sacrifice of experimental animals is necessary. However, in the case of molecular imaging, because evaluation is carried out by images using imaging reporter genes, there is little or no damage to experimental animals, and because it is possible to carry out repeated experiments in one individual, it is possible to trace the change in genetic expression according to time using images. Namely, the molecular imaging method has problems in that, living body tissue is not damaged, repeated experiments in one individual can be carried out, and it is possible to carry out quantitative analysis through imaging.

Among imaging diagnostic methods, the use of magnetic resonance imaging (MRI) that is the newest diagnostic method is being increased. MRI is basically to measure signals from protons. MRI aimed at noninvasive diagnosis at first, but since an MRI contrast agent was first marketed in 1988, it has been found that the use of MRI contrast agents can improve diagnostic sensitivity and specificity. Recently, the application of MRI contrast agents has been gradually enlarged to MRI angiography, perfusion imaging and the like.

Such MRI contrast agents are classified according to their effects on a magnetic field into paramagnetic and superparamagnetic materials which are called "positive contrast agents" and "negative contrast agents", respectively. The former has a predominant T1 shortening effect, and thus is expressed as a light signal in T1-weighted images, and the latter has a predominant T2 shortening effect, and thus is expressed as a dark signal in T2-weighted images. As the superparamagnetic materials, iron oxides are used, and they may be classified according to particle size into two categories: superparamagnetic iron oxide (SPIO) having a particle size of more than 50 nm, and ultrasmall superparamagnetic iron oxide (USPIO) having a particle size of less than 50 nm. In the case in which the superparamagnetic materials are applied, negative contrast is required, and a signal in a portion to which the contrast agent has been delivered is reduced so as to make the image darker than that of the surrounding tissue (Susanne, M. et al., Invest. Radio., 37:167, 2002).

Iron oxide contrast agents which are currently being mainly studied are SPIO-based colloids which are either nanosized magnetite or magnetite coated with polymeric carbohydrate such as dextran or starch. Magnetic nanoparticles are nanoparticles in a colloidal iron oxide solution and are used as contrast agents because of the large relaxation signal of the nanoparticles. The size of nanoparticles in the iron oxide solution has various distributions depending on the size and coating thickness of the core thereof and the pattern of aggregation thereof (Gro, C. et al., Cells Mater., 3:163, 2002). Gd contrast agents show an excellent contrast effect at a concentration of a few millimoles, whereas SPIO shows an excellent contrast effect even at a concentration of a few nanomoles, and thus can be studied and applied as MRI contrast agents (Bulte, J.W. et al., Res. Med., 25:148, 1992; Knauth, M. et al., Am. J. Neuroradiol., 22:99, 2001; Lacava, L.M. et al., Biophys., 252:367, 2002). For use as contrast agents, it is necessary to prepare a magnetic solution which has high saturation magnetization and, at the same time, small, uniform and stable. Pure magnetic particles are not used because of the following limitations: (1) they have a high aggregation propensity; (2) if they are not sufficiently stable, the inherent structure thereof can change, leading to a change in the magnetic properties thereof; (3) when they come into contact with a biological environment, they can be rapidly biodegraded; and (4) they are toxic by themselves. To reduce such problems, polymer-coated SPIO and USPIO have been developed (Lind, K. et al., J. Drug Target., 10:221, 2002; Bellin, M.F. et al., J. Radiol., 34:257, 2000; Bonnemain, B. et al., Review. J. Drug Target., 6:167, 1998; Muller, R.N. et al., Invest Radiol., 25:34, 1990).

However, in order to use polymer-coated magnetic nanoparticles in the cell imaging and biomedical fields, a targeted drug delivery approach is required in which a drug is delivered only to target cells or lesions within a short time so as to treat or destroy the target cells or tissues. According to a previous report, in an experiment conducted before applying stem cell therapy to clinical trials, when stem cells and other mammalian cells were labeled with magnetic nanoparticles in order to examine the cell penetration of the nanoparticles *in vitro,* the magnetic nanoparticles were not efficient, because a long culture time was required for the magnetic particles to be translocated into the cells (Frank, J.A. et al., Academic Radiology, 484:487, 2002).

Accordingly, there has been a need for methods which effectively delivery biologically active micromolecules both *in vivo* and *in vitro* without damaging cells. Examples of such methods include a method of chemically adding lipid peptides or a method which uses basic polymers such as polylysine or polyarginine, but these methods have not yet been verified. It was reported that folate acid which is used as a transporter is delivered into cells in the form of a folate conjugate (Leamon, C.P. et al., Proc. Natl. Acd. Sci., 88:5572, 1991), but whether folate is delivered into the cytoplasm has not yet been verified. Furthermore, *Pseudomonas* exotoxin is also used as a kind of transporter (Prior, T.I. et al., Cell, 64:1017, 1991). However, the effects of these methods on the intracellular migration of physiologically activated substances and their general applicability thereof have not yet been clearly verified. Accordingly, there has been a continuous demand for a method capable of delivering a biologically active substance into the cytoplasm or nucleus of living cells in a safer and more effective way.

As a result of studies on this demand, cell penetrating peptides (CPPs) have been suggested. Among these, TAT protein, which is a transcription factor of human immunodeficiency virus-1, HIV-1) has been mostly well studied. It has been found that the Tat protein was more effective in passing through the cell membrane, when it consisted of amino acids 47 to 57 (YGRKKRRQRRR), on which positively charged amino acids were concentrated, than when it was in the form of a full-length 86 amino acid protein (Fawell, S. et al., Proc. Natl. Acad. Sci. USA, 91:664, 1994). Other examples of cell penetrating peptides (CPPs) include a peptide having an amino acid sequence consisting of amino acids 267 to 300 of the VP22 protein of HSV-1 (herpes simplex virus type 1) (Elliott, G. et al., Cell, 88:223, 1997), a peptide having an amino acid sequence consisting of amino acids 84 to 92 of the UL-56 protein of HSV-2 (GenBank code:D1047 [gi:221784]), and a peptide having an amino acid sequence consisting of amino acids 339 to 355 of the antennapedia (ANTP) protein of *Drosophila sp.* (Schwarze, S.R. et al., Trends. Pharmacol. Sci., 21:45, 2000). In addition, artificial peptides consisting of positively charged amino acids were also found to be effective (Laus R. et al., Nature Biotechnol. 18:1269-1272 (2000)).

Meanwhile, since it was found that, when CPP is fused with other peptides or proteins, the intracellular delivery of such fusion proteins is effective, various applications of PTD have been attempted (Korean Patent Registration No. 10-0568457). However, the cell penetrating peptide has a problem in the terms of safety, because it is derived from virus.

As a peptide which has a sequence similar to that of TAT and is used as a cell penetrating domain, low molecular weight protamine (LMWP) containing a large amount of cationic amino acids such as arginine etc. was recently reported. Particularly, LMWP is a naturally derived cationic peptide derived from protamine and has advantages in that it has no risk of toxicity and can be produced in large amounts.

FUNOVICS MARTIN ET AL., "Nanoparticles for the optical imaging of tumor E-selectin", NEOPLASIA, NEOPLASIA PRESS, ANN ARBOR, MI, US, vol. 7, no. 10, 1 October 2005, pages 904-911, XP009105232, ISSN: 1522-8002, DOI: 10.1593/NEO.05352, discloses a fluorescent peptide-magnetic nanoparticle conjugate that images E-selectin expression in mouse xenograft models of Lewis lung carcinoma (LLC) by fluorescence reflectance imaging.

US 2006/251726 A1 (LIN JACKI ET AL.), 9 November 2006, corresponding to WO 2007/149062 A2, discloses a nanoparticle-polypeptide complex comprising a bioactive polypeptide in association with a nanoparticle, wherein the bioactive polypeptide is modified by the addition of a chemical moiety that facilitates cellular uptake of the protein.

MARCELO J KOGAN ET AL., "Peptides and metallic nanoparticles for biomedical applications", NANOMEDICINE, vol. 2, no. 3, 1 June 2007, pages 287-306, XP55104016, ISSN: 1743-5889, DOI: 10.2217/17435889.2.3.287, describes the contribution of peptides to the biomedical applications of metallic nanoparticles.

JOSEPHSON L ET AL., "High-efficiency intracellular magnetic labeling with novel superparamagnetic-Tat peptide conjugates", BIOCONJUGATE CHEMISTRY, ACS, WASHINGTON, DC, US, vol. 10, no. 2, 1 February 1999, pages 186-191, XP002172120, ISSN: 1043-1802, DOI: 10.1021/BC980125H, discloses the derivatization of a biocompatible, dextran coated superparamagnetic iron oxide particle with a peptide sequence from the HIV-tat protein to improve intracellular magnetic labeling of different target cells. The conjugate had a mean particle size of 41 nm and contained an average of 6.7 tat peptides. Derivatized particles were internalized into lymphocytes over 100-fold more efficiently than non-modified particles, resulting in up to 12.7 x 10⁶ particles/cell.

WO 2005/107818 A2, (UNIVERSITY FLORIDA), 17 November 2005, discloses fluorescent, radio-opaque and magnetic quantum nanoparticles, useful as multifunctional contrast agents or probes for *in vivo* bioimaging, and methods of their use.

US 7 037 652 B2 (WYETH), 2 May 2006, discloses methods for detecting, characterizing, preventing and treating prostate cancer by providing KIAA markers wherein changes in the levels of expression of one or more of the KIAA markers is correlated with the presence of prostate cancer.

US 7 019 113 B2 (MIRUS BIO CORPORATION), 28 MARCH 2006, discloses a process for the reversible modification of membrane interaction of a compound to facilitate delivery of molecules to cells *in vivo* and *in vitro.*

US 6 867 186 B2 (NEW ENGLAND MEDICAL CENTER HOSPITAL), 15 March 2005, discloses fusion proteins that contain a calpastatin peptide and a signal sequence capable of delivering the fusion protein into a cell.

WO 2004/081188 A2 (UNIVERSITY MICHIGAN), 23 September 2004, discloses compositions for transport across a biological membrane including a membrane-translocating LMWP peptide and a cargo molecule.

PARK YOON YEONG ET AL., "Nontoxic membrane translocation peptide from protamine, low molecular weight protamine (LMWP), for enhanced intracellular protein delivery: in vitro and in vivo study", THE FASEB JOURNAL, FEDERATION OF AMERICAN SOCIETIES FOR EXPERIMENTAL BIOLOGY, BETHESDA, US, vol. 19, no. 9, 1 July 2005, pages 1-20, XP002587876, ISSN: 0892-6638, DOI: 10.1096/FJ.04-2322FJE, discloses the preparation of low molecular weight protamines by enzymatic digestion of protamine, the examination of their capability of transducing an impermeable protein toxin into the tumor cells by chemical conjugation, and the determination of the cytotoxicity of transduced protein toxin against cancer cell lines and a tumor-bearing mouse. It was shown that LMWPs could indeed translocate themselves into several mammalian cell lines as efficiently as TAT, thereby transducing impermeable gelonin into the cells by chemical conjugation.

APOSTOLOPOULOS V ET AL., "Delivery of tumor associated antigens to antigen presenting cells using penetratin induces potent immune responses", VACCINE, ELSEVIER LTD, GB, vol. 24, no. 16, 12 April 2006, pages 3191-3202, XP028010607, ISSN. 0264-410x, DOI. 10:1016/J.VACCINE.2006.01.032, discloses that Int, incorporating MUC1 CTL epitopes in tandem is able to facilitate their rapid uptake by macrophages and dendritic cells (DC) in an energy-dependent endocytic pathway. It was also demonstrated that Int conjugated proteins are also able to be efficiently taken up by DC.

CHRISTIAENS B ET AL., "Enhancement of polymethacrylate-mediated gene delivery by Penetratin", EUROPEAN JOURNAL OF PHARMACEUTICAL SCIENCES, ELSEVIER, AMSTERDAM, NL, vol. 24, no. 5, 1 April 2005, pages 525-537, XP027803775, ISSN: 0928-0987, discloses the combination of polymethacrylates with Penetratin, a 16-residue water-soluble peptide that internalizes into cells through membrane translocation to increase the transfection efficiency of DNA.

Therefore, there is an urgent need to develop a technology for imaging diagnosis which shows high safety and maximizes the effect of imaging diagnosis through optimal targeting, unlike existing viral peptide transporters, but the results of studies thereon are still insignificant.

Accordingly, the present inventors have made many efforts to solve the above-described problems occurring in the prior art and, as a result, have found that, when a cell-penetrating peptide is chemically linked to a fluorescence-labeled magnetic nanoparticle, the magnetic nanoparticle can be stably delivered into cells without a process of endocytosis, thereby completing the present invention.

### SUMMARY OF INVENTION

It is an object of the present invention to provide a cell-penetrating peptide/fluorescence-labeled magnetic nanoparticle complex in which the cell-penetrating peptide is linked to the fluorescence-labeled magnetic nanoparticle.

Another object of the present invention is to provide a composition for cell imaging containing said complex.

To achieve the above objects, the present invention provides a method for preparing a cell-penetrating peptide/fluorescence-labeled magnetic nanoparticle complex, the method comprising the steps of: (a) preparing an amino group-containing magnetic nanoparticle by modifying the surface of magnetic nanoparticle with an amino group; (b) preparing a fluorescence-labeled magnetic nanoparticle by adding a fluorescent substance to the amino group-containing magnetic nanoparticle; and (c) preparing cell-penetrating peptide/fluorescence-labeled magnetic nanoparticle complex by linking a cell-penetrating peptide to the fluorescence-labeled magnetic nanoparticle, wherein the diameter of said complex is 10-50 nm and wherein the cell penetrating peptide is low molecular weight protamine (LMWP, SEQ ID NO:1: VSRRRRRRGGRRRR).

The present invention also provides a complex of a cell-penetrating peptide and a fluorescence-labeled magnetic nanoparticle, which is prepared according to said method and in which the cell-penetrating peptide is linked to the fluorescence-labeled magnetic nanoparticle wherein the diameter of said complex is 10-50 nm; and a composition for cell imaging containing said complex, in which the cell-penetrating peptide is linked to the fluorescence-labeled magnetic nano-particle as defined above.

Other features and embodiments of the present invention will be more apparent from the following detailed description and the appended claims.

### BRIEF DESCRIPTION OF DRAWINGS

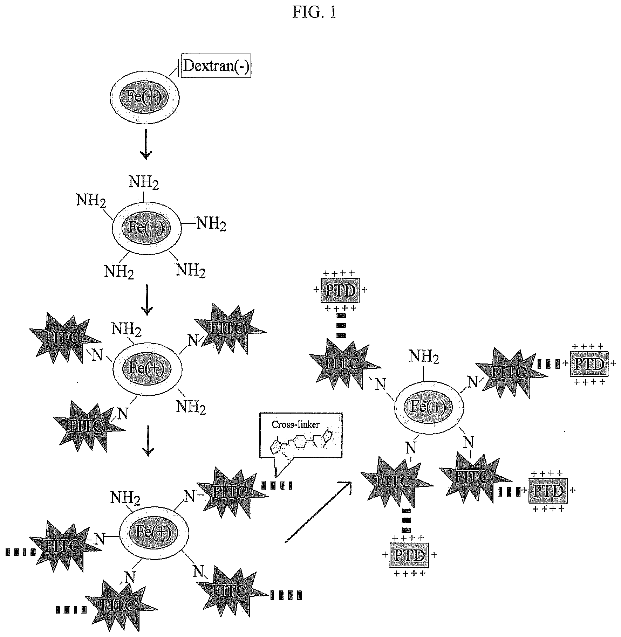

FIG. 1 schematically shows a method for preparing fluorescence-labeled magnetic nanoparticle/cell-penetrating peptide complex according to the present invention.

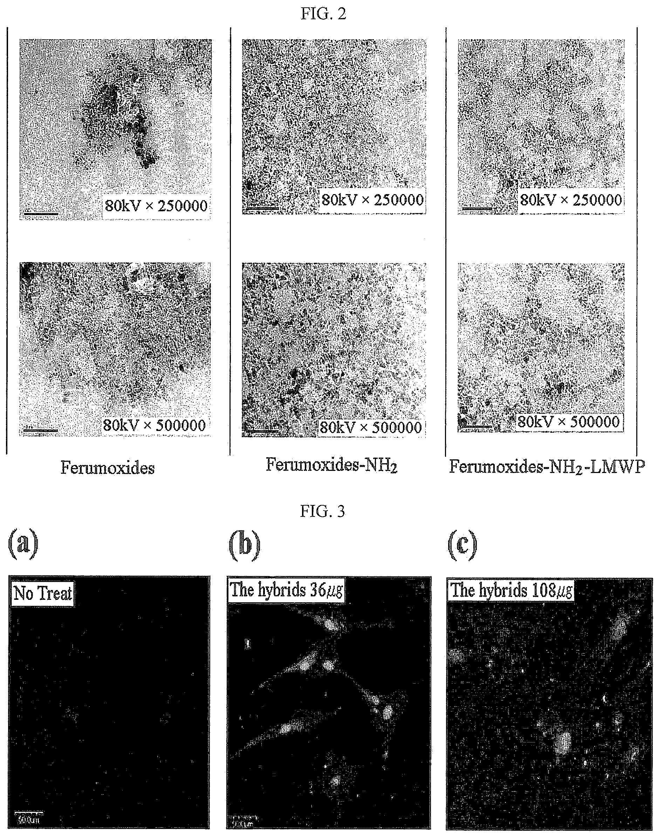

FIG. 2 depicts transmission electron microscope (TEM) photographs showing the dispersion of fluorescence-labeled magnetic nanoparticle/cell-penetrating peptide complex, magnetic nanoparticle and amino group-containing magnetic nanoparticle in water. In FIG. 2, (a) and (d): photographs at 250,000x and 500,000x of magnetic nanoparticles dispersed in water; (b) and (e): photographs at 250,000x and 500,000x of amino group-containing magnetic nanoparticles dispersed in water; and (c) and (f): photographs at 250,000x and 500,000x of fluorescence-labeled magnetic nanoparticle/cell-penetrating peptide complex.
FIG. 3 depicts confocal scanning micrographs showing the cell penetration of fluorescence-labeled magnetic nanoparticle/cell-penetrating peptide complex according to the present invention at varying concentrations [(a): 0 µg of the fluorescence-labeled magnetic nanoparticle/cell-penetrating peptide complex; (b): 36 µg of the fluorescence-labeled magnetic nanoparticle/cell penetrating peptide complex; and (c): 108 µg of the fluorescence-labeled magnetic nanoparticle/cell-penetrating peptide complex].

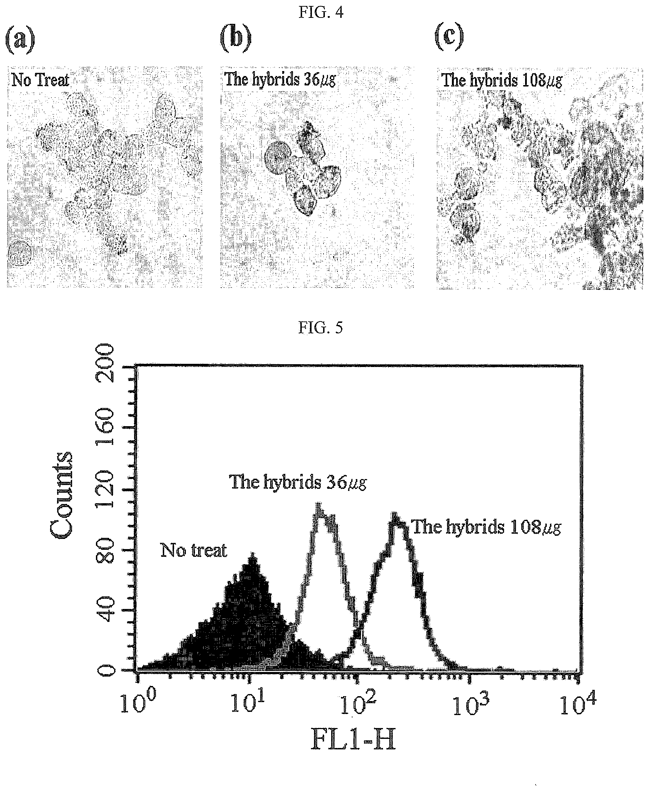

FIG. 4 depicts optical micrographs showing the cell penetration of fluorescence-labeled magnetic nanoparticle/cell-penetrating peptide complex according to the present invention at varying concentrations [(a): 0 µg of the fluorescence-labeled magnetic nanoparticle/cell-penetrating peptide complex; (b): 36 *µ*g of the fluorescence-labeled magnetic nanoparticle/cell penetrating peptide complex; and (c): 108 *µ*g of the fluorescence-labeled magnetic nanoparticle/cell-penetrating peptide complex].
FIG. 5 is a graphic diagram showing the results of flow cytometric analysis showing the cell penetration of fluorescence-labeled magnetic nanoparticle/cell-penetrating peptide complex according to the present invention at varying concentrations.

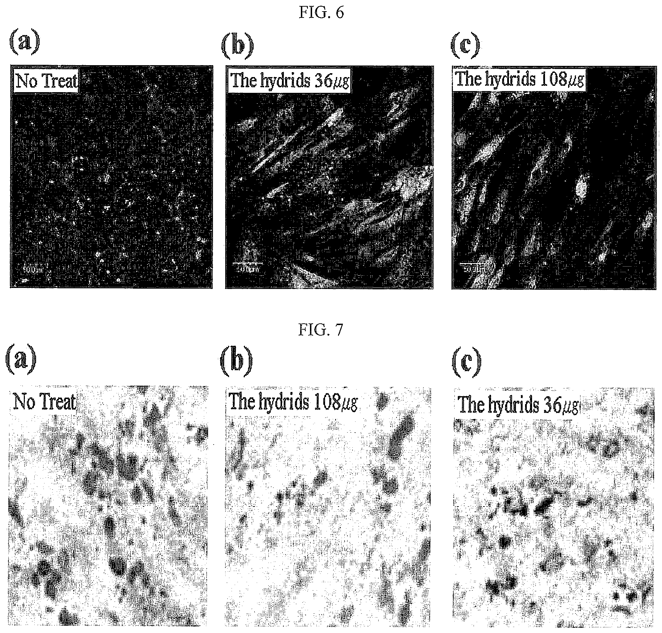

FIG. 6 depicts confocal scanning micrographs showing the osteoblast differentiation of fluorescence-labeled magnetic nanoparticle/cell-penetrating peptide complex according to the present invention at varying concentrations [(a): 0 *µ*g of the fluorescence-labeled magnetic nanoparticle/cell-penetrating peptide complex; (b): 36 *µ*g of the fluorescence-labeled magnetic nanoparticle/cell penetrating peptide complex; and (c): 108 *µ*g of the fluorescence-labeled magnetic nanoparticle/cell-penetrating peptide complex].
FIG. 7 depicts optical micrographs showing the osteoblast differentiation of fluorescence-labeled magnetic nanoparticle/cell-penetrating peptide complex according to the present invention at varying concentrations [(a): 0 *µ*g of the fluorescence-labeled magnetic nanoparticle/cell-penetrating peptide complex; (b): 36 *µ*g of the fluorescence-labeled magnetic nanoparticle/cell penetrating peptide complex; and (c): 108 *µ*g of the fluorescence-labeled magnetic nanoparticle/cell-penetrating peptide complex].

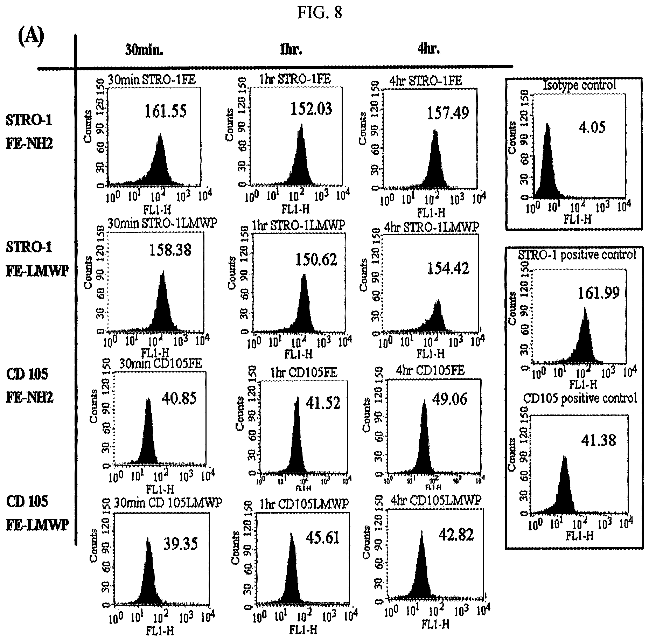

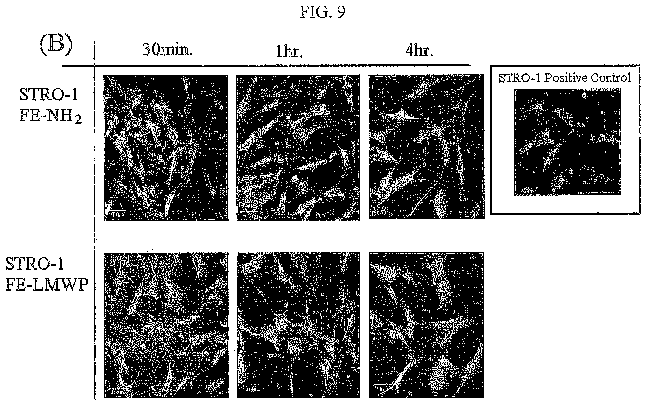

FIG. 8 and FIG. 9 show measurement results for the effect of a fluorescence-labeled magnetic nanoparticle-cell penetrating peptide complex according to the present invention on mesenchymal stem cells. Specifically, FIG. 8 is a graphic diagram showing the results of flow cytometry for mesenchymal stem cells as model cells treated with fluorescence-labeled antibodies, STRO-1 and CD105, and FIG. 9 depicts confocal scanning micrographs of mesenchymal stem cells as model cells treated with a fluorescence-labeled antibody, STRO-1.

### DETAILED DESCRIPTION OF THE INVENTION, AND PREFERRED EMBODIMENTS

In one aspect, the present invention relates to a method for preparing a complex of a cell-penetrating peptide and a fluorescence-labeled magnetic nanoparticle, the method comprising the steps of: (a) preparing an amino group-containing magnetic nanoparticle by modifying the surface of magnetic nanoparticle with an amino group; (b) preparing a fluorescence-labeled magnetic nanoparticle by adding a fluorescent substance to the amino group-containing magnetic nanoparticle; and (c) linking a cell-penetrating peptide to the fluorescence-labeled magnetic nanoparticle, wherein the diameter of said complex is 10-50 nm and wherein the cell-penetrating peptide is low molecular weight protamine (LMWP; SEQ ID NO: 1: VSRRRRRRGGRRRR).

Cell-penetrating peptide/fluorescence-labeled magnetic nanoparticle according to the present invention is prepared by the chemical interaction between a cell-penetrating peptide (CPP) serving as a transporter and fluorescence-labeled magnetic nanoparticles enabling the imaging diagnosis of target cells and tissues. When the cell-penetrating peptide/magnetic nanoparticle complex is introduced in vivo in the form of an aqueous solution, they penetrate directly into cells without an endocytosis process, show high stability, maximize imaging diagnosis through optimal targeting and minimize side effects, unlike existing viral peptide transporters.

As used herein, the term "magnetic nanoparticle" means superparamagnetic material that can temporarily interact with the proton of the surrounding water. Preferably, it means magnetic iron oxide nanoparticle having a particle size of 10-150 nm. More preferably, it means MRI contrast agents that enable the images of substantially all the cells and organs of the body to be obtained. The magnetic nanoparticle is preferably selected from the group consisting of ferric ammonium citrate (FAC), ferrum oxide, superparamagnetic iron oxide nanoparticle (SPIO), carboxy-dextran coated iron oxide nanoparticle, ultrasmall superparamagnetic iron oxide nanoparticle (USPIO), monocrystalline iron oxide nanoparticles (MION), polycrystalline iron oxide nanoparticles (PION) and oral magnetic particles (OMP).

Also, the fluorescent substance that is used in the present invention is preferably fluorescein isothiocyanate (FITC) or tetramethylrhodamine isothiocyanate (TRITC), but is not limited thereto and may include all chemically modified fluorescent substances, for example alexa fluor, rhodamine red-X, texas red, tetramethylrhodamine, cascade blue, DAPI(4',6-diamidino-2-phenylindole), coumarine, lucifer yellow, dansylaminde, etc.

The cell-penetrating peptide is low molecular weight protamine (LMWP, SEQ ID NO: 1; VSRRRRRRGGRRRR). The cell-penetrating peptide/magnetic nanoparticle complex according to the present invention may be prepared either by linking the peptide and the nanoparticles to each other through the electrostatic interaction between the positive charge of amino acid and the negative charge of dextran coated on the magnetic nanoparticles or by inducing the chemical linkage there between using a crosslinker. In a preferred embodiment of the present invention, the complex may be prepared by modifying the surface of the magnetic nanoparticles to make a reactive group on the surface in order to increase stability in an *in vivo* environment, and then inducing the chemical linkage between the magnetic nanoparticles and the peptide using a crosslinker. Because the cell-penetrating peptide that is used in the present invention has free amino groups at the terminal end, it is easy to form the complex using the crosslinker.

The crosslinker that is used in the present invention may be selected from the group consisting of, but not limited to, 1,4-bis-maleimidobutane (BMB), 1,11-bis-maleimidotetraethyleneglycol (BM[PEO]4), 1-ethyl-3-[3-dimethyl aminopropyl] carbodiimide hydrochloride (EDC), succinimidyl-4-[N-maleimidomethylcyclohexane-1-carboxy-[6-amidocaproate]] (SMCC) and sulfonate thereof (sulfo-SMCC), succimidyl 6-[3-(2-pyridyldithio)-ropionamido] hexanoate (SPDP) and sulfonate thereof (sulfo-SPDP), m-maleimidobenzoyl-N-hydroxysuccinimide ester (MBS) and sulfonate thereof (sulfo-MBS), succimidyl[4-(p-maleimidophenyl)butyrate] (SMPB) and sulfonate (sulfo-SMPB) thereof.

As shown in FIG. 1, the cell-penetrating peptide/magnetic nanoparticle complex according to the present invention is prepared by modifying the surface of negatively charged dextran-coated magnetic nanoparticles with ammonium hydroxide to prepare amino group-containing magnetic nanoparticles, allowing the amino group-containing magnetic nanoparticles to react with a fluorescent substance so as to prepare fluorescence-labeled magnetic nanoparticles, and then adding a crosslinker thereto to prepare fluorescence-labeled magnetic nanoparticles having a free sulfhydryl group. Meanwhile, the cell-penetrating peptide may be coated with a crosslinker to prepare a cell-penetrating peptide having a free sulfhydryl group, which may be allowed to react with said fluorescence-labeled magnetic nanoparticles having a free sulfhydryl group, thus a cell-penetrating peptide/fluorescence-labeled magnetic nanoparticle complex.

In the preparation of the cell-penetrating peptide/fluorescence-labeled magnetic nanoparticle complex according to the present invention, modifying the surface of the magnetic nanoparticles with an amino group may be carried out using one selected from the group consisting of ammonium hydroxide, ethanol, aminopropyl trimethylsilane (APTMS), N-(3-dimethylpropyl)-N-ethylcarbodiimide hydrochloride (EDC), N-hydroxysulfosuccinimide (sulfo-NHS) and N-hydroxysuccinimide (NHS).

In the cell-penetrating peptide/magnetic nanoparticle complex according to the present invention, the cell-penetrating peptide and the magnetic nanoparticles are linked by S-S bonds. Accordingly, the magnetic nanoparticles may be dissociated from the cell-penetrating peptide by reductase and etc. present in cells, and thus the magnetic nanoparticles may be introduced into cells in an easy and simple way, such that cell imaging and diagnosis can be easily carried out.

In another aspect, the present invention relates to a complex of a cell-penetrating peptide and a fluorescence-labeled magnetic nanoparticle; and a composition for cell imaging containing said complex, wherein the diameter of said complex is 10-50 nm.

The composition for cell imaging according to the present invention is preferably administered parenterally, for example, via bolus injection, intravenous injection or intraarterial injection or spray (e.g., aerosol spray) for contrast of lungs. In addition, the composition may be administered via the oral or rectal route and may also be administered according to any known method for administering contrast agents. When the composition according to the present invention is administered parenterally, it must be administered in an aseptic state and must be free of a physiologically unacceptable agent and a paramagnetic, superparamagnetic, ferromagnetic or ferromagnetic contaminant. The composition according to the present invention may contain a preservative, an antimicrobial agent, a buffer solution that is conventionally used in parenteral solutions, an antioxidant, excipient, and other additives that can be used in combination with MR contrast agents and do not interfere with the preparation, storage or use of products.

Also, although the dosage of the composition for cell imaging according to the present invention will vary depend on the target tissue or organ used and the properties of a measurement system, it is preferably kept at a level as low as possible, as long as sensible contrast can be achieved. The dosage may generally be in the range corresponding to LD₅₀ 10%, that is, 1-1000 mg/kg, preferably 2-500 mg/kg, and more preferably 3-300 mg/kg.

The cell-penetrating peptide/magnetic nanoparticle complex prepared by linking the magnetic nanoparticles for cell imaging and tracing with the cell-penetrating peptide according to the present invention were analyzed for their cell penetration ability and their effect on the inherent characteristics of cells. As a result, it could be observed that the cell penetration of the cell-penetrating peptide/magnetic nanoparticle complex was increased in a concentration-dependent manner. When cells were cultured in various conditions and stained, the complex had no effect on the differentiation of human mesenchymal stem cells (hMSC) into osteoblasts and adipocytes. In addition, it could be observed that, even when model cells were treated with high concentrations of the cell-penetrating peptide/magnetic nanoparticle complex using a cell marker that is predominantly expressed in the cells, the inherent characteristics of the cells did not change.

Furthermore, the cell-penetrating peptide/magnetic nanoparticle complex was measured for their particle size and surface potential. As a result, it was shown that the magnetic nanoparticle complex had a uniform particle size distribution of 10-50 nm, such that they could penetrate even into compact tissue. In addition, it was shown that the surface potential of magnetic nanoparticles was increased through linkage to positively charged amino groups, suggesting that the process of linking the cell-penetrating peptide to the magnetic nanoparticles was performed in an accurate way.

### Examples

Hereinafter, the present invention will be described in further detail with reference to examples.

Particularly, although the following examples illustrated only ferumoxides, iron oxide contrast agents, as magnetic nanoparticles, it will be obvious to a person skilled in the art that the same as or similar effects to the case of ferumoxides can be obtained even when ferric ammonium citrate (FAC), ferrum oxide, superparamagnetic iron oxide nanoparticle (SPIO), carboxy-dextran coated iron oxide nanoparticle, ultrasmall superparamagnetic iron oxide nanoparticle (USPIO), monocrystalline iron oxide nanoparticles (MION), polycrystalline iron oxide nanoparticles (PION), oral magnetic particles (OMP), etc. are used for the imaging diagnosis of cells and organs.

### Example 1: Preparation of cell-penetrating peptide/fluorescence-labeled magnetic nanoparticle complex

### 1-1: Preparation of fluorescence-labeled magnetic nanoparticle

2 ml (11.2 mg/ml) of magnetic nanoparticles (AMI; Cambridge, MA, USA) coated with positively charged dextran was added to 0.5 ml of ammonium hydroxide (Sigma, USA), 10 ml of sodium hydroxide (Sigma, USA), 4 ml of triple deionized water and 4 ml of epichlorohydrin (Sigma, USA). The mixture was allowed to react at room temperature for 24 hours, and then dialyzed with triple deionized water to remove unreacted ammonium hydroxide. To the solution from which unreacted ammonium hydroxide has been removed, 5 mM sodium citrate was added, so that the alkaline environment of the solution was adjusted to a neutral environment. The solution was concentrated using an ultrafiltration membrane (MWCO 3,000) so as to remove unbound reactants. Then, a solution of 3 mg of fluorescent substance FITC (fluorescein isothiocyanate) or TRITC (tetramethylrhodamine isothiocyanate) in 120 *µ*ℓ of N,N-dimethylformamide (DMF) was added thereto and reacted under stirring at 25°C.

### 1-2: Preparation of cell-penetrating peptide/fluorescence-labeled magnetic nanoparticle complex

14 mg of an SMCC crosslinker (Pierce Biotechnology) was dissolved in 0.5 ml of DMSO (dimethyl sulfoxide), and then 6 ml of the fluorescence-labeled magnetic nanoparticle solution prepared in Example 1-1 was added thereto. The mixture solution was stirred at room temperature for 5 hours, and then ultrafiltered twice with alkaline phosphate buffer saline (PBS, pH 8). To 6 ml of the ultrafiltered solution containing the fluorescence-labeled magnetic nanoparticles, the cell-penetrating peptide, low molecular weight protamine (LMWP (VSRRRRRRGGRRRR)), dissolved in 1 ml of phosphate buffer saline (PBS, 10X, pH 8.4) at a concentration of 5 mM, was added, and the mixture was stirred at room temperature for 2 hours and at 4°C for 24 hours, thus linking the cell-penetrating peptide (low molecular weight peptide) with the fluorescence-labeled magnetic nanoparticles. The linked low molecular weight protamine and fluorescence-labeled magnetic nanoparticles were ultrafiltered twice with triple deionized water, and the pH of the linked material was measured with pH-measuring paper.

### Comparative Example 1: General magnetic nanoparticles

Magnetic nanoparticles (AMI, Cambridge, MA, USA) coated with negatively charged dextran were purchased and used.

### Comparative Example 2: Preparation of amino group-containing magnetic nanoparticles

Amino group-containing magnetic nanoparticles were prepared according to the same composition and method as in Example 1-1, except that the step of adding the fluorescent substance was excluded.

### Experimental Example 1: Measurement of particle size and distribution pattern of cell-penetrating peptide/fluorescence-labeled magnetic nanoparticle complex

Each of the cell-permeable peptide/fluorescence-labeled magnetic nanoparticle complex prepared in Example 1 and, as controls, the magnetic nanoparticles of Comparative Example 1 and the amino group-containing magnetic nanoparticles of Comparative Example 2, was dissolved in water. Each of the solutions was dropped onto a carbon-coated copper grid in amounts of 2 mg/mℓ and 10 *µ*ℓ, and then naturally dried for 20 hours. The size of each of the dried particles and the dispersion thereof in water were observed with a transmission electron microscope (TEM, JEM-2000EXII, JEOL, JAPAN) at 250,000x magnification and 500,000x magnification at a voltage of 80 kV.

As a result, as shown in FIG. 2, the cell-penetrating peptide/fluorescence-labeled magnetic nanoparticle complexes (c) and (f) showed uniform particle distribution similar to the magnetic nanoparticles (a) and (d) and the amino group-containing magnetic nanoparticles (b) and (e). Also, the particle size determined based on the scale bar was 10-35 nm and similar between the three groups. This suggests that there is no great change in particle size and distribution pattern during the process of chemically linking the cell-permeable peptide with the fluorescence-labeled magnetic nanoparticles and that the cell-penetrating peptide/fluorescence-labeled magnetic nanoparticle complex may be used in contrast agents, because the magnetic nanoparticles have a small size of less than 50 nm and show uniform distribution.

### Experimental Example 2: Measurement of particle size and surface potential of cell-penetrating peptide/fluorescence-labeled magnetic nanoparticle complex

To measure the numerical value of the particle size of the cell-penetrating peptide/fluorescence-labeled magnetic nanoparticle complex, a particle size analyzer (PSSNICOMP, US) was used. The cell-penetrating peptide/fluorescence-labeled magnetic nanoparticle complex prepared in Example 1, the magnetic nanoparticles prepared of Comparative Example 1 and the amino group-containing magnetic nanoparticles of Comparative Example 2 were dissolved in water at a concentration of 2 mg/ml and placed into a glass cuvette, and then the particle size thereof was measured at 23 °C and 632.8 nm.

Also, to measure the numerical value of the surface potential of the cell-permeable peptide/fluorescence-labeled magnetic nanoparticle complex, a zeta potential analyzer (Otsuka, Japan) was used. The cell-penetrating peptide/fluorescence-labeled magnetic nanoparticle complex prepared in Example 1, the magnetic nanoparticles prepared of Comparative Example 1 and the amino group-containing magnetic nanoparticles of Comparative Example 2 were dissolved in water at a concentration of 2 mg/ml and placed into the analyzer, and then the surface potential thereof was measured.

As a result, as shown in Table 1 below, the cell-permeable peptide/fluorescence-labeled magnetic nanoparticle complex had a particle size of 26.8 nm which was not greatly different from the particle size (20.4 nm) of the magnetic nanoparticles of Comparative Example 1 and the particle size (23.8 nm) of the amino group-containing magnetic nanoparticles of Comparative Example 2. Also, the magnetic nanoparticle complex had a particle size distribution of less than 50 nm, such that they could penetrate even into compact tissue. Furthermore, the surface potential of the particles was measured to be -51.54 for the magnetic nanoparticles of Comparative Example 1, -10.72 for the amino group-containing magnetic nanoparticles of Comparative Example 2, and 16.97 for the cell-penetrating peptide/fluorescence-labeled magnetic nanoparticle complex of Example 1. Thus, it could be seen that, when the negatively charged dextran-coated magnetic nanoparticles were linked with the positively charged amino group and the cell-penetrating peptide LMWP, the surface potential was changed to positive charges. In addition, it could be seen that the process of preparing the magnetic nanoparticle/LMWP complex was performed in an accurate way.

**Table 1: Measurement of particle size and surface potential**

| | Particle size (nm) | Surface potential (mV) |
|---|---|---|
| Magnetic nanoparticle (ferumoxides) | 20.4 | -51.54 |
| Amino group-containing magnetic nanoparticle (ferumoxides-NH₂) | 23.8 | -10.72 |
| Cell-permeable peptide/fluorescence-labeled magnetic nanoparticle complex (ferumoxides-NH₂-LMWP) | 26.8 | 16.97 |

### Experimental Example 3: Measurement of in vitro cell permeability of cell-penetrating peptide/fluorescence-labeled nanoparticle complex

### 3-1: Measurement of cell permeability

In order to measure the cell penetration of the cell-penetrating peptide/fluorescence-labeled magnetic nanoparticles, human mesenchymal stem cells (hMSC) were seeded in each well of a 4-well chamber slide at a density of 1 x 10⁴ cells/well, and then incubated in general medium for 20 hours (overnight). Then, the fluorescence-labeled magnetic nanoparticle/cell-penetrating peptide complex (prepared in Example 1) was added to FBS (fetal bovine serum)-free medium in each well in varying concentrations of 0 *µ*g, 36 *µ*g and 108 *µ*g, and after 1 hour, each well was washed twice with phosphate buffer saline (PBS). Then, the cells were stained with a nuclear staining dye (Hoechst 33342, blue), and the cell penetration of the cell-penetrating peptide/magnetic nanoparticle complex was observed with a confocal scanning microscope (IX 70, Olympus Co., Tokyo, Japan).

As a result, as shown in FIG. 3, when the cells were treated with each of 0 *µ*g, 36 *µ*g and 108 *µ*g of the cell-penetrating peptide/fluorescence (FITC, green)-labeled magnetic nanoparticle complex prepared in Example 1, fluorescent particles were observed in the cytoplasm and nucleus of the cells treated with 36 *µ*g of the cell-penetrating peptide/fluorescence-labeled magnetic nanoparticle complex (b) and 108 *µ*g of the cell-penetrating peptide/fluorescence-labeled magnetic nanoparticle complex (c), and the cell penetration of the cell-penetrating peptide/fluorescence-labeled magnetic nanoparticle complex was increased in a concentration-dependent manner. Also, it was seen that the magnetic nanoparticle had no effect on the inherent delivery ability of the cell-penetrating peptide.

### 3-2: Measurement of intracellular permeability using iron oxide staining

In order to examine the intracellular penetration of the fluorescence-labeled magnetic nanoparticle/cell-penetrating peptide complex, human mesenchymal stem cells (hMSC) were cultured in a 6-well plate. Before carrying out Prussian blue staining (Perl's reagent staining), the fluorescence-labeled magnetic nanoparticle/cell-penetrating peptide complex prepared in Example 1 were added to the culture medium in varying amounts of 0 *µ*g, 36 *µ*g and 108 *µ*g, followed by incubation for 20 hours (overnight). After completion of the incubation, the cells were separated from each well using 0.25% trypsin, and the cells were dispended onto a cytospin slide at a density of 2 x 10⁵ cells. The dispensed cells were fixed with 10% NBF (neutral buffered formalin), and then washed twice with phosphate buffer saline (PBS). The iron oxide molecules which penetrated into the cells were stained blue with 2% Perl's reagent, and then the cells were stained red with nuclear fast red. After mounting the slide, the intracellular penetration of the fluorescence-labeled magnetic nanoparticle/cell-penetrating peptide complex was observed with an optical microscope (CKX41, Olympus Co., Tokyo, Japan).

As a result, as shown in FIG. 4, it could be seen that, in the case of 36 *µ*g of the fluorescence-labeled magnetic nanoparticle/cell-penetrating peptide complex (b) and 108 *µ*g of the fluorescence-labeled magnetic nanoparticle/cell-penetrating peptide complex (c), the magnetic nanoparticles were stained (blue) in the cells, and that the intracellular delivery of the magnetic nanoparticles was increased with an increase in the concentration of the fluorescence-labeled magnetic nanoparticle/cell-penetrating peptide complex.

### 3-3: Measurement of cell penetration ability using flow cytometry

In order to quantitatively analyze the cell penetration of the fluorescence-labeled magnetic nanoparticle/cell-penetrating peptide complex, human mesenchymal stem cells (hMSC) were plated in each well of a 6-well plate at a density of 1 x 10⁵ cells/well, and then incubated in general medium for 20 hours (overnight). The cell-penetrating peptide/fluorescence (FITC)-labeled magnetic nanoparticle complex prepared in Example 1 were added to a fetal bovine serum (FBS)-free DMEM medium in each well in varying amounts of 0 *µ*g, 36 *µ*g and 108 *µ*g*.* After 1 hour, each well was washed twice with phosphate buffer saline (PBS), and the cells were separated from each well using 0.25% trypsin, washed with phosphate buffer saline (PBS), and then centrifuged to remove the supernatant. The washing and centrifugation procedure was repeated twice, thus removing the fluorescent dye outside the cells. The cells were suspended in 1 mℓ of phosphate buffer saline (PBS), and then the intracellular delivery of the fluorescence-labeled magnetic nanoparticle/cell-penetrating peptide complex of Example 1 were observed using FACSCalibur (BD, USA) at FL-1 (488 nm).

As a result, as shown in FIG. 5, in the case of 36 *µ*g of the cell-penetrating peptide/fluorescence-labeled magnetic nanoparticle complex (b) and 108 *µ*g of the cell-penetrating peptide/fluorescence-labeled magnetic nanoparticle complex (c), the number of cells containing fluorescent particles was increased with an increase in the concentration of complex used. This suggests that the cell penetration ability of the complex was increased in proportion to the increase in the concentration of the cell-penetrating peptide.

### Experimental Example 4: Effect of cell-penetrating peptide/fluorescence-labeled magnetic nanoparticle complex on in vitro differentiation of mesenchymal stem cells

### 4-1: Differentiation into osteoblasts

In order to examine the effect of the cell-penetrating peptide/fluorescence-labeled magnetic nanoparticle complex on the differentiation of cells, human mesenchymal stem cells (hMSC) were seeded in a 4-well chamber slide at a density of 5 x 10³ density/well, and then incubated in general medium for 20 hours (overnight) to stabilize the cells. The cell-penetrating peptide/fluorescence (TRITC, red)-labeled magnetic nanoparticle complex prepared in Example 1 were added to each well in varying amounts of 0 *µ*g*,* 36 *µ*g and 108, and then incubated for 24 hours. After completion of the incubation, the cells were cultured in a hard tissue-forming medium containing calcein (calcium green) for 14 days. The composition of the hard tissue-forming medium consisted of alpha-MEM medium containing 15% FBS (fetal bovine serum), 50 mg/mℓL-ascorbic acid, 10⁻⁷ M dexamethasone, 1% antibiotic-antimycotic solution and 10 mM beta-glycerol phosphate. After completion of the culture, the medium was removed, and then the cells were washed twice with phosphate (PBS). The washed cells were fixed with 10% NBF (neutral buffered formalin) and stained with a nuclear staining dye (Hoechst 33342, blue), and whether the magnetic nanoparticles having the cell-penetrating peptide linked thereto are involved in differentiation into osteoblasts was observed with a confocal scanning microscope (IX 70, Olympus Co., Tokyo, Japan).

As a result, as shown in FIG. 6, in the case of 36 *µ*g of the cell-penetrating peptide/fluorescence-labeled magnetic nanoparticle complex (b) and 108 *µ*g of the cell-penetrating peptide/fluorescence-labeled magnetic nanoparticle complex (c), calcein staining was uniformly distributed as compared to the group (a) not treated with the cell-penetrating peptide/fluorescence-labeled magnetic nanoparticle complex, and red intracellular staining was observed. This suggests that the cell-penetrating peptide/fluorescence-labeled magnetic nanoparticle complex penetrated into the cells, but had no effect on the osteoblast differentiation of the model cells, even when they were used at high concentration (108 *µ*g).

### 4-2: Adipocyte differentiation of mesenchymal stem cells

Human mesenchymal stem cells (hMSC) were plated in a 6-well plate at a density of 1 x 10⁵ cells/well and incubated for 20 hours (overnight) to stabilize the cells. Then, the cell-penetrating peptide/fluorescence-labeled magnetic nanoparticle complex prepared in Example 1 were added to each well in varying concentrations of 0 *µ*g*,* 36 *µ*g and 108 *µ*g and incubated for 24 hours. Then, the cells were incubated for 3 days in an adipocyte differentiation-inducing medium containing insulin, a hormone involved in lipid metabolism, and indomethacin, a substance promoting the synthesis of arachidonic acid that is a kind of fatty acid, and then, the cells were incubated for 2 days in a maintenance medium containing medium supplements other than insulin. This cell incubation procedure was repeated three times, and then the cells were incubated in a maintenance medium for 7 days to induce differentiation into adipocytes. The differentiation-induced cells were fixed with 10% NBF (neutral buffered formalin), washed with 60% isopropanol and completely dried. Then, fat in the cells was stained with oil red O solution (Sigma, red), and each well was washed with water (H₂O). Then, whether the magnetic nanoparticles having the cell-penetrating peptide linked thereto are involved in differentiation into adipocytes was observed with an optical microscope.

As a result, as shown in FIG. 7, the cells treated with each of 36 *µ*g of the cell-penetrating peptide/fluorescence-labeled magnetic nanoparticle complex (b) and 108 *µ*g of the cell-penetrating peptide/fluorescence-labeled magnetic nanoparticle complex (c) showed oil red O staining (red: index of adipocyte differentiation) in the same manner as the group (c) not treated with the cell-penetrating peptide/fluorescence-labeled magnetic nanoparticle complex. This suggests that the cell-penetrating peptide/fluorescence-labeled magnetic nanoparticle complex had no effect on differentiation into adipocytes, even when they were used at high concentration (108 *µ*g)*.*

### Experimental Example 5: Effect of cell-penetrating peptide/fluorescence-labeled magnetic nanoparticle complex on inherent characteristics of mesenchymal stem cells

### 5-1: Numerical expression of effect on inherent characteristics of mesenchymal stem cells

In order to examine the effect of the cell-penetrating peptide/fluorescence-labeled magnetic nanoparticle complex on the inherent characteristics of mesenchymal stem cells, mouse anti-human endoglin (CD 105) monoclonal antibody (CHEMICON, USA) and mouse anti-STRO-1 monoclonal antibody (CHEMICON, USA), which are expressed predominantly in human mesenchymal stem cells (hMSC), were used as primary antibodies, and the goat anti-mouse IgG (H&L) fluorescein-conjugated affinity-purified secondary antibody (CHEMICON, USA) was used (Puchuantes, S. et al., Tissue Antigens, 50:6, 1997). Human mesenchymal stem cells (hMSC) were plated in each well of a well plate at a density of 1 x 10⁶ cells/well, and then incubated in a general medium for 20 hours. Then, 36 *µ*g/mℓ of the cell-penetrating peptide/fluorescence-labeled magnetic nanoparticle complex (prepared in Example 1) in DMEM medium containing no FBS (fetal bovine serum) were added to each well and incubated for 30 minutes, 1 hour and 4 hours. Then, each well was washed twice with phosphate buffer saline (PBS), and the cells were separated from each well using 0.25% trypsin and washed with phosphate buffer saline. The washed cells were centrifuged to remove the supernatant. The centrifugation procedure was repeated twice to remove particles outside the cells. Then, the primary antibodies, Endoglin (CD 105) and STRO-1, diluted in bovine serum albumin (BSA) at a ratio of 1:25, were added to the cells, and then phosphate buffer saline (PBS) was added thereto. Then, the solution was incubated under shaking at 4 °C for 20 hours. The incubated solution was washed with 0.5% bovine serum albumin (BSA)-containing phosphate buffer saline (PBS), and then centrifuged to remove the supernatant. The secondary antibody diluted in 0.5% bovine serum albumin (BSA)-containing phosphate buffer saline (BSA) at a ratio of 1:50 was added thereto, and then the solution was incubated under shaking at room temperature for 1 hour. Then, the cells were suspended in 300 *µ*ℓ of phosphate buffer saline (PBS), and the intracellular delivery of the cell-penetrating peptide/fluorescence-labeled magnetic nanoparticle complex was observed using FACSCalibur (BD, USA) at FL-1 (488 nm).

As a result, as shown in FIG. 8, when the fluorescent dye (FITC, green)-conjugated hMSC (human mesenchymal stem cell) marker, STRO-1 and Endoglin (CD 105) were allowed to react with the model cells labeled with the cell-penetrating peptide/magnetic nanoparticle complex, the fluorescence value observed in the model cells was similar to that of the positive control. This suggests that the cell-penetrating peptide/magnetic nanoparticle complex has no effect on the inherent characteristics of human mesenchymal stem cell (hMSC) as the model cells. Also, this indirectly reveals that the cell-penetrating peptide/magnetic nanoparticle complex according to the present invention has no cytotoxicity when they are used for cell imaging.

### 5-2: Visualization of effect on inherent characteristics of mesenchymal stem cells

In order to examine the effect of the cell-penetrating peptide/magnetic nanoparticle complex on the inherent characteristics of mesenchymal stem cells, human mesenchymal stem cells (hMSC) were seeded in a 4-well chamber slide at a density of 5 x 10³ cells/well, and then incubated in general medium for 20 hours (overnight) to stabilize the cells. Then, 36 *µ*g/mℓ of the cell-penetrating peptide/magnetic nanoparticle complex in DMEM medium containing no FBS (fetal bovine serum) were added to each well and incubated for 30 minutes, 1 hour and 4 hours. Then, each well was washed with phosphate buffer saline (PBS), and the cells were fixed with 10% NBF (neutral buffered formalin) and stained with a nuclear staining dye (Hoechst 33342, blue). Then, the primary antibodies, Endoglin (CD 105) and STRO-1, diluted in 0.5% bovine serum albumin (BSA)-containing phosphate buffer saline (PBS) at a ratio of 1:25, were added thereto and incubated under shaking at 4°C for 20 hours (overnight). The incubated solution was washed with 0.5% bovine serum albumin (BSA)-containing phosphate buffer saline (PBS), and the secondary antibody diluted in 0.5% bovine serum albumin-containing phosphate buffer saline (PBS) at a ratio of 1:50 was added thereto. Then, the solution was incubated under shaking at room temperature for 1 hour. The incubated solution was washed with phosphate buffer saline (PBS). Then, the chamber slide was mounted, and the cells were observed with a confocal scanning microscope.

As a result, as shown in FIG. 9, when the fluorescent dye (FITC, green)-conjugated hMSC (human mesenchymal stem cell) marker, STRO-1 and Endoglin (CD 105) were allowed to react with the model cells labeled with the cell-penetrating peptide/magnetic nanoparticle complex, the fluorescence value observed in the model cells was similar to that of the positive control. This suggests that the cell-penetrating peptide/magnetic nanoparticle complex has no effect on the inherent characteristics of human mesenchymal stem cell (hMSC) as the model cells. Also, this indirectly reveals that the cell-penetrating peptide/magnetic nanoparticle complex according to the present invention has no cytotoxicity when they are used for cell imaging.

### INDUSTRIAL APPLICABILITY

As described above in detail, unlike the existing viral peptide transporters, the present invention can suggest an innovative therapeutic technology which shows high stability, maximizes the effect of imaging diagnosis through optimal targeting and minimizes side effects.

## Claims

1. A method for preparing a complex of a cell-penetrating peptide and a fluorescence-labeled magnetic nanoparticle, the method comprising the steps of:
(a) preparing an amino group-containing magnetic nanoparticle by modifying the surface of magnetic nanoparticle with an amino group;
(b) preparing a fluorescence-labeled magnetic nanoparticle by adding a fluorescent substance to the amino group-containing magnetic nanoparticle; and
(c) preparing cell-penetrating peptide/fluorescence-labeled magnetic nanoparticle complex by linking a cell-penetrating peptide to the fluorescence-labeled magnetic nanoparticle, wherein the diameter of said complex is 10-50nm and
wherein the cell-penetrating peptide is low molecular weight protamine (LMWP, SEQ ID NO: 1 : VSRRRRRRGGRRRR).

2. The method for preparing a complex of a cell-penetrating peptide and a fluorescence-labeled magnetic nanoparticle according to claim 1, wherein the magnetic nanoparticle is selected from the group consisting of ferric ammonium citrate (FAC), ferrum oxide, superparamagnetic iron oxide nanoparticle (SPIO), carboxy-dextran coated iron oxide nanoparticle, ultrasmall superparamagnetic iron oxide nanoparticle (USPIO), monocrystalline iron oxide nanoparticles (MION), polycrystalline iron oxide nanoparticles (PION) and oral magnetic particles (OMP).

3. The method for preparing a complex of a cell-penetrating peptide and a fluorescence-labeled magnetic nanoparticle according to claim 1, wherein said linking of step (c) is performed by a crosslinker.

4. The method for preparing a complex of a cell-penetrating peptide and a fluorescence-labeled magnetic nanoparticle according to claim 3, wherein the crosslinker is selected from the group consisting of 1,4-bis-maleimidobutane (BMB), 1,11-bis-maleimidotetraethyleneglycol (BM[PEO]4), 1-ethyl-3-[3-dimethyl aminopropyl] carbodiimide hydrochloride (EDC), succinimidyl-4-[N-maleimidomethylcyclohexane-1-carboxy-[6-amidocaproate]] (SMCC) and sulfonate thereof (sulfo- SMCC), succimidyl 6-[3-(2-pyridyldithio)-ropionamido] hexanoate (SPDP) and sulfonate thereof (sulfo-SPDP), m-maleimidobenzoyl-N-hydroxysuccinimide ester (MBS) and sulfonate thereof (sulfo-MBS), succimidyl[4-(p-maleimidophenyl)butyrate] (SMPB) and sulfonate (sulfo-SMPB) thereof.

5. The method for preparing a complex of a cell-penetrating peptide and a fluorescence-labeled magnetic nanoparticle according to claim 1, wherein the fluorescent substance is selected from the group consisting of fluorescein isothiocyanate (FITC) or tetramethylrhodamine isothiocyanate (TRITC), alexa fluor, rhodamine red-X, texas red, tetramethylrhodamine, cascade blue, DAPI(4',6-diamidino-2-phenylindole), coumarine, lucifer yellow, and dansylaminde.

6. The method for preparing a complex of a cell-penetrating peptide and a fluorescence-labeled magnetic nanoparticle according to claim 1, wherein said modifying the surface of the magnetic nanoparticle of step (a) is carried out using one selected from the group consisting of ammonium hydroxide, ethanol, aminopropyl trimethylsilane (APTMS), N-(3-dimethylpropyl)-N-ethylcarbodiimide hydrochloride (EDC), N-hydroxysulfosuccinimide (sulfo-NHS) and N-hydroxysuccinimide (NHS).

7. The method for preparing a complex of a cell-penetrating peptide and a fluorescence-labeled magnetic nanoparticle according to claim 1, which further comprising the step of adjusting pH to neutral by adding sodium citrate to the amino group-containing magnetic nanoparticle prepared in step (a).

8. A complex of a cell-penetrating peptide and a fluorescence-labeled magnetic nanoparticle, which is prepared by the method of claim 1, and in which the cell-penetrating peptide is linked to the fluorescence-labeled magnetic nanoparticle, wherein the diameter of said complex is 10-50nm.

9. A composition for cell imaging containing the complex in which the cell-penetrating peptide is linked to the fluorescence-labeled magnetic nanoparticle of claim 8.

## Patentansprüche

1. Verfahren zur Herstellung eines Komplexes aus einem zellpenetrierenden Peptid und einem fluoreszenzmarkierten magnetischen Nanoteilchen, wobei das Verfahren die Stufen umfasst:
(a) Herstellen eines Aminogruppen-enthaltenden magnetischen Nanoteilchens durch Modifizieren der Oberfläche des magnetischen Nanoteilchens mit einer Aminogruppe;
(b) Herstellen eines fluoreszenzmarkierten magnetischen Nanoteilchens durch Zugabe einer fluoreszierenden Substanz zu dem Aminogruppen-enthaltenden magnetischen Nanoteilchen und
(c) Herstellen eines Komplexes aus einem zellpenetrierenden Peptid/fluoreszenzmarkierten magnetischen Nanoteilchen durch Verknüpfen eines zellpenetrierenden Peptids mit dem fluoreszenzmarkierten magnetischen Nanoteilchen, wobei der Durchmesser des Komplexes 10-50 nm beträgt und
wobei das zellpenetrierende Peptid ein niedrigmolekulares Protamin (LMWP, SEQ ID NO: 1 : VSRRRRRRGGRRRR) ist.

2. Verfahren zur Herstellung eines Komplexes aus einem zellpenetrierenden Peptid und einem fluoreszenzmarkierten magnetischen Nanoteilchen nach Anspruch 1, wobei das magnetische Nanoteilchen aus der Gruppe, bestehend aus Eisenammoniumcitrat (FAC), Eisenoxid, superparamagnetischem Eisenoxidnanoteilchen, Kohlenstoffdextran-beschichteten Eisenoxidnanoteilchen, ultrakleinen superparamagnetischen Eisenoxidnanoteilchen (USPIO), monokristallinen Eisenoxidnanoteilchen (MION), polykristallinen Eisenoxidnanoteilchen (PION) und oralen magnetischen Teilchen (OMP) ausgewählt ist.

3. Verfahren zur Herstellung eines Komplexes aus einem zellpenetrierenden Peptid und einem fluoreszenzmarkierten magnetischen Nanoteilchen nach Anspruch 1, wobei die Verknüpfung von Stufe (c) durch einen Crosslinker durchgeführt wird.

4. Verfahren zur Herstellung eines Komplexes aus einem zellpenetrierenden Peptid und einem fluoreszenzmarkierten magnetischen Nanoteilchen nach Anspruch 3, wobei der Crosslinker ausgewählt ist aus der Gruppe, bestehend aus 1,4-Bis-maleimidobutan (BMB), 1,11-Bis-maleimidotetraethylenglycol (BM[PEO]4), 1-Ethyl-3-[3-dimethylaminopropyl]carbodiimid-Hydrochlorid (EDC), Succinimidyl-4-[N-maleimidomethylcyclohexan-1-carboxy-[6-amidocaproat]] (SMCC) und Sulfonat davon (Sulfo-SMCC), Succimidyl-6-[3-(2-pyridyldithio)-ropionamido]hexanoat (SPDP) und Sulfonat davon (Sulfo-SPDP), m-Maleimidobenzoyl-N-hydroxysuccinimidester (MBS) und Sulfonat davon (Sulfo-MBS), Succimidyl[4-(p-maleimidophenyl)butyrat] (SMPB) und Sulfonat (Sulfo-SMPB) davon.

5. Verfahren zur Herstellung eines Komplexes aus einem zellpenetrierenden Peptid und einem fluoreszenzmarkierten magnetischen Nanoteilchen nach Anspruch 1, wobei die fluoreszierende Substanz ausgewählt ist aus der Gruppe, bestehend aus Fluoresceinisothiocyanat (FITC) oder Tetramethylrhodaminisothiocyanat (TRITC), Alexafluor, Rhodamin Red-X, Texas Red, Tetramethylrhodamin, Cascade Blue, DAPI (4',6-Diamidino-2-phenylindol), Coumarin, Lucifergelb und Dansylamid.

6. Verfahren zur Herstellung eines Komplexes aus einem zellpenetrierenden Peptid und einem fluoreszenzmarkierten magnetischen Nanoteilchen nach Anspruch 1, wobei das Modifizieren der Oberfläche des magnetischen Nanoteilchens von Stufe (a) unter Verwendung einer Verbindung, ausgewählt aus der Gruppe, bestehend aus Ammoniumhydroxid, Ethanol, Aminopropyltrimethylsilan (APTMS), N-(3-Dimethylpropyl)-N-ethylcarbodiimid-Hydrochlorid (EDC), N-Hydroxysulfosuccinimid (Sulfo-NHS) und N-Hydroxysuccinimid (NHS) durchgeführt wird.

7. Verfahren zur Herstellung eines Komplexes aus einem zellpenetrierenden Peptid und einem fluoreszenzmarkierten magnetischen Nanoteilchen nach Anspruch 1, umfassend weiter die Stufe des Einstellens des pH-Wertes auf neutral durch Zugabe von Natriumcitrat zu dem in Stufe (a) hergestellten Aminogruppen-enthaltenden magnetischen Nanoteilchen.

8. Komplex aus einem zellpenetrierenden Peptid und einem fluoreszenzmarkierten magnetischen Nanoteilchen, das nach dem Verfahren nach Anspruch 1 hergestellt wurde und worin das zellpenetrierende Peptid an das fluoreszenzmarkierte magnetische Nanoteilchen geknüpft ist, wobei der Durchmesser des Komplexes 10-50 nm beträgt.

9. Zusammensetzung zur Zellabbildung, enthaltend den Komplex in dem das zellpenetrierende Peptid an das fluoreszenzmarkierte magnetische Nanoteilchen nach Anspruch 8 gebunden ist.

## Revendications

1. Procédé de préparation d'un complexe d'un peptide pénétrant les cellules et d'une nanoparticule magnétique à marquage fluorescent, le procédé comprenant les étapes suivantes :
(a) préparation d'une nanoparticule magnétique contenant un groupe amino par modification de la surface de la nanoparticule magnétique avec un groupe amino ;
(b) préparation d'une nanoparticule magnétique à marquage fluorescent par ajout d'une substance fluorescente à la nanoparticule magnétique contenant un groupe amino ; et
(c) préparation d'un complexe peptide pénétrant les cellules/nanoparticule magnétique à marquage fluorescent par liaison d'un peptide pénétrant les cellules à la nanoparticule magnétique à marquage fluorescent, dans lequel le diamètre dudit complexe est de 10 à 50 nm et
dans lequel le peptide pénétrant les cellules est une protamine de poids moléculaire bas (LMWP, SEQ ID NO : 1 : VSRRRRRRGGRRRR).

2. Procédé de préparation d'un complexe d'un peptide pénétrant les cellules et d'une nanoparticule magnétique à marquage fluorescent selon la revendication 1, dans lequel la nanoparticule magnétique est sélectionnée dans le groupe constitué du citrate d'ammonium ferrique (FAC), de l'oxyde ferreux, d'une nanoparticule d'oxyde de fer superparamagnétique (SPIO), d'une nanoparticule d'oxyde de fer revêtue de carboxy-dextrane, d'une nanoparticule d'oxyde de fer superparamagnétique ultra-petite (USPIO), de nanoparticules d'oxyde de fer monocristallin (MION), de nanoparticules d'oxyde de fer polycristallin (PION) et de particules magnétiques orales (OMP).

3. Procédé de préparation d'un complexe d'un peptide pénétrant les cellules et d'une nanoparticule magnétique à marquage fluorescent selon la revendication 1, dans lequel ladite liaison de l'étape (c) est réalisée par un agent de réticulation.

4. Procédé de préparation d'un complexe d'un peptide pénétrant les cellules et d'une nanoparticule magnétique à marquage fluorescent selon la revendication 3, dans lequel l'agent de réticulation est sélectionné dans le groupe constitué du 1,4-bis-maléimidobutane (BMB), du 1,11-bis-maléimidotétraéthylèneglycol (BM[PEO]4), du chlorhydrate de 1-éthyl-3-[3-diméthylaminopropyl]carbodiimide (EDC), du 4-[N-maléimidométhylcyclohexane-1-carboxy-[6-amidocaproate]] de succinimidyle (SMCC) et du sulfonate de celui-ci (sulfo-SMCC), du 6-[3-(2-pyridyldithio)-ropionamido]hexanoate de succimidyle (SPDP) et du sulfonate de celui-ci (sulfo-SPDP), d'un ester de m-maléimidobenzoyl-N-hydroxysuccinimide (MBS) et du sulfonate de celui-ci (sulfo-MBS), du [4-(p-maléimidophényl)butyrate] de succimidyle (SMPB) et du sulfonate de celui-ci (sulfo-SMPB).

5. Procédé de préparation d'un complexe d'un peptide pénétrant les cellules et d'une nanoparticule magnétique à marquage fluorescent selon la revendication 1, dans lequel la substance fluorescente est sélectionnée dans le groupe constitué de l'isothiocyanate de fluorescéine (FITC) ou de l'isothiocyanate de tétraméthylrhodamine (TRITC), d'Alexa Fluor, de la rhodamine Red-X, du Texas Red, de la tétraméthylrhodamine, du Cascade Blue, du DAPI (4',6-diamidino-2-phénylindole), de la coumarine, du jaune lucifer, et du dansylaminde.

6. Procédé de préparation d'un complexe d'un peptide pénétrant les cellules et d'une nanoparticule magnétique à marquage fluorescent selon la revendication 1, dans lequel ladite modification de la surface de la nanoparticule magnétique de l'étape (a) est réalisée en utilisant l'un sélectionné dans le groupe constitué de l'hydroxyde d'ammonium, de l'éthanol, de l'aminopropyle triméthylsilane (APTMS), du chlorhydrate de N-(3-diméthylpropyl]-N-éthylcarbodiimide (EDC), du N-hydroxysulfosuccinimide (sulfo-NHS) et du N-hydroxysuccinimide (NHS).

7. Procédé de préparation d'un complexe d'un peptide pénétrant les cellules et d'une nanoparticule magnétique à marquage fluorescent selon la revendication 1, comprenant en outre l'étape d'ajustement du pH à neutre par l'ajout de citrate de sodium à la nanoparticule magnétique contenant un groupe amino préparée dans l'étape (a).

8. Complexe d'un peptide pénétrant les cellules et d'une nanoparticule magnétique à marquage fluorescent, qui est préparé par le procédé selon la revendication 1, et dans lequel le peptide pénétrant les cellules est lié à la nanoparticule magnétique à marquage fluorescent, dans lequel le diamètre dudit complexe est de 10 à 50 nm.

9. Composition pour imagerie cellulaire contenant le complexe dans lequel le peptide pénétrant les cellules est lié à la nanoparticule magnétique à marquage fluorescent selon la revendication 8.


### SEQUENCE LISTING

<110> Seoul National University Industry Foundation
<120> Complex of Cell Translocational Peptide and Magnetic Nanoparticulates and
   Use Thereof
<130> 21275EP
<140> EP 08 835 082.2
   <141> 2008-10-02
<150> 10-2007-0099511
   <151> 2007-10-02
<160> 4
<170> PatentIn version 3.2
<210> 1
   <211> 14
   <212> PRT
   <213> Unknown
<400> 1
   val Ser Arg Arg Arg Arg Arg Arg Gly Gly Arg Arg Arg Arg 14
<210> 2
   <211> 11
   <212> PRT
   <213> Artificial Sequence
<220>
   <223> TAT (TAT from protein transduction domain of HIV protein)
<400> 2
   Tyr Gly Arg Lys Lys Arg Arg Gln Arg Arg Arg 11
<210> 3
   <211> 16
   <212> PRT
   <213> Artificial Sequence
<220>
   <223> penetratin
<400> 3
   Arg Gln Ile Lys lie Trp Phe Gln Asn Arg Arg Met Lys Trp Lys Lys 16
<210> 4
   <211> 8
   <212> PRT
   <213> unknown
<400> 4
   Arg Arg Arg Arg Arg Arg Arg Arg 8